# EUROPEAN PATENT SPECIFICATION

(11) **EP 0 758 237 B1**
(45) Date of publication and mention of the grant of the patent: **27.08.2003**
(21) Application number: 95917929.2
(22) Date of filing: 18.04.1995
(51) Int. Cl.: A61K 31/4745, A61K 9/08, A61K 9/107, A61K 47/10

(54) **LACTONE STABLE FORMULATION OF CAMPTOTHECIN OR 7-ETHYL CAMPTOTHECIN IN DIMETHYLISOSORBIDE OR DIMETHYLACETAMIDE**
LACTONE-STABILE CAMPTOTHECIN ODER 7 - ETHYL CAMPTOTHECIN - FORMULIERUNG IN DIMETHYLISOSORBID ODER DIMETHYLACETAMID
FORMULATION STABLE SOUS FORME LACTONE DE CAMPTOTHECINE OU 7-ETHYLE CAMPTOTHECINE DANS DU DIMETHYLISOSORBIDE OU DU DIMETHYLACETAMIDE

(30) Priority: 28.04.1994 US 234131; 09.05.1994 US 240035
(43) Date of publication of application: 19.02.1997
(73) Proprietor: BIONUMERIK PHARMACEUTICALS, INC., San Antonio, Texas 78229 (US)
(72) Inventor: HAUSHEER, Frederick, Herman, San Antonio, TX 78209 (US); HARIDAS, Kochat, San Antonio, TX 78240 (US)
(74) Representative: Lucas, Brian Ronald
(86) International application number: EP9501432
(87) International publication number: WO95029677

(56) References cited:
- EP-A- 0 359 184
- US-A- 3 219 529
- US-A- 5 004 758
- US-A- 5 124 318
- PROCEEDINGS OF THE AMERICAN ASSOCIATION FOR CANCER RESEARCH, vol. 35, March 1994 page 245 RUBIN ET AL 'A Phase I trial of 9-amino-camptothecin'

## Description

### BACKGROUND OF THE INVENTION

### 1. FIELD OF THE INVENTION

Camptothecin ("CPT") and 7-ethyl camptothecin ("ECPT") are known to have anti-tumour activity. However, the E-ring lactone forms of these compounds are poorly soluble in water. Because of its poor water solubility, the lactone form has not been directly administered by parenteral or oral routes in human subjects for the purpose of inhibiting the growth of cancer cells. Hydrolysis of the lactone to the carboxylate form greatly increases water-solubility but significantly reduces the anti-tumour activity. This invention overcomes these limitations and claims novel pharmaceutically acceptable formulations of CPT or ECPT, and antitumor compositions comprising them, both per se and for use in human anti-cancer therapy.

### 2. DESCRIPTION OF THE RELATED ART

A water soluble derivative of CPT, 7-ethyl-10-[4-(1-piperidino)-1-piperidino] carbonyloxycamptothecin ("CPT-11") has anti-tumour activity due to metabolic conversion thereof to its active metabolite 10-hydroxy-7-ethyl camptothecin (HECPT). The metabolic conversion varies from patient to patient and limits the utility of CPT-11 in achieving the highest plasma concentrations of HECPT which can be tolerated by the patient. HECPT's poor solubility in water has previously made the direct administration of HECPT impractical for the treatment of cancer. The conversion of CPT-11 to HECPT involves a putative carboxyl esterase enzyme, which is believed to be mainly responsible for the metabolic production of HECPT from CPT-11. Human lung cancer cell lines have been observed to convert less CPT-11 to HECPT than normal cells. The cancer cells' decreased metabolic conversion represents a form of resistance to CPT-11 and limits the utility of CPT-11 in terms of reliably and safely achieving adequate plasma concentrations of HECPT to inhibit the growth of cancer cells in humans.

Each of CPT, ECPT and HECPT exists in two forms which are in equilibrium in vivo, namely a lactone ring form (1a) and an open, carboxylate form (1b). These isomeric forms are inter-convertible: a reversible pH-dependent hydrolysis converts the lactone ring form, produced in acid conditions, to the open, carboxylate form produced in alkaline conditions. The equilibrium is shown below: 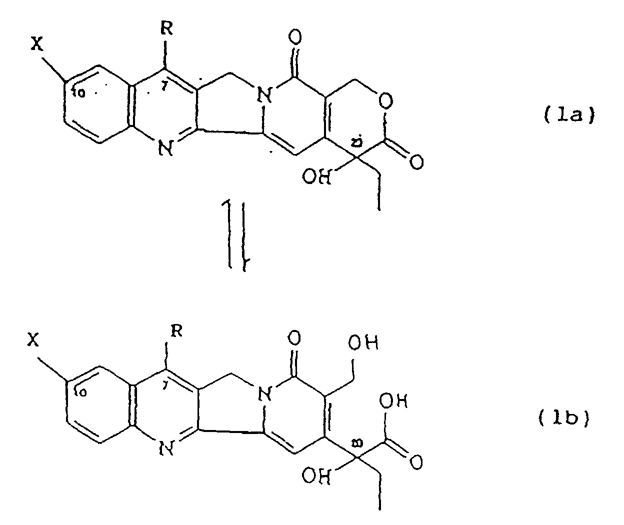
CPT: R=X=H
ECPT: R=C₂H₅, X=H
HECPT: R=C₂H₅, X=HO

CPT, ECPT and HECPT are considered to derive their activity by inhibiting the enzyme topoisomerase I, which is involved in DNA replication: it relieves the torsional strain introduced ahead of the moving replication fork. Only the lactone ring form inhibits topoisomerase I. See M.L. Rothenberg et al., Journal of Chemical Oncology, 11, 2194 -2204 (November 1993).

It has been a problem that CPT, ECPT and HECPT have been considered unsuitable for direct clinical use because their most active forms are poorly soluble in water.

Ruben et al, Proceedings of the American Association for Cancer Research, vol. 35, March 1994, page 245, discloses a Phase I trial of 9-amino-camptothecin (9-AC). 9-AC was administered to patients as a continuous infusion in polyethylene glycol and DMA. There is no disclosure that the 9-AC as administered was completely dissolved in this formulation.

In relation specifically to HECPT, the invention of our prior PCT application WO 9517187 solved this problem by providing a stabilized formulation of the lactone form HECPT, therein called "lactone stable HECPT" for brevity. The formulation of that invention is a solution or suspension comprising (1) HECPT, (2) dimethylisosorbide (DMI) or dimethylacetamide (DMA) and (3) a pharmaceutically acceptable acid.

### SUMMARY OF THE INVENTION

It has now been found that CPT and ECPT can likewise be stabilised in the same way. The formulation of the present invention is an acidic solution comprising (1) CPT or ECPT, more than 50 mole % of which is in the lactone form, and (2) DMI or DMA. Such a formulation is herein likewise called "lactone stable".

Several schedules and various dosages produce sufficient levels of lactone stable CPT or ECPT to yield beneficial antitumor effects in humans. The effective levels of CPT or ECPT are reasonably safe in terms of the incidence and severity of specific side effects that may occur with administration and are acceptable within standard medical practice for patients undergoing treatment for cancer.

Direct administration of lactone stable CPT or ECPT is likely to offer several important clinical advantages over administration of CPT-11. For example:
1. it allows the clinician to tailor the administration of the active cytoxic species to suit the patient's tolerance;
2. it overcomes interpatient variability which may be due to polymorphism of key enzyme(s) in the metabolism of CPT-11 to HECPT; and
3. clinicians can more consistently optimize the drug dosage and schedule to achieve the maximum tolerated dose of CPT or ECPT which is likely to lead to the most beneficial clinical anti-cancer effect.

Regarding the clinical utility of CPT or ECPT for the treatment of human cancer, this invention provides the following:
1. lactone stable CPT or ECPT;
2. lactone stable CPT or ECPT for administration to patients with cancer;
3. antitumor compositions comprising lactone stable CPT or ECPT;
4. lactone stable CPT or ECPT for parenteral administration;
5. lactone stable CPT or ECPT for administration according to pharmacologic schedules for achieving the maximum tolerated dose with acceptable clinical toxicity observed in standard clinical practice of cancer treatment;
6. lactone stable CPT or ECPT for oral administration;
7. lactone stable CPT or ECPT for the treatment of localized complications of cancer by direct administration via instillation into various body cavities.

### DESCRIPTION OF THE PREFERRED EMBODIMENTS

Dimethylisosorbide (DMI) has been used as solvent for muscle relaxants (U.S. Pat. No. 3,699,230), tetracyclines (U.S. Pat. No. 3,219,529), aspirin (U.S. Pat. No. 4,228,162), and steroids (U.S. Pat. No. 4,082,881). Dimethylacetamide (DMA) has been proposed as a solvent for 5-azacytosine arabinoside (ara-c) and 5-azacytidine at pH 7.0 to 8.3: see U.S. Patent 4,684,630 (Repta). DMI and DMA have very good toxicity profiles and are miscible with ethanol, propylene glycol, isopropyl myristate, water, diethyl ether, corn oil, acetone, cottonseed oil, and the like.

The formulations of the invention are solutions. They will be liquids, for the purpose of convenient pharmaceutical administration and handling appropriate to the routes of administration mentioned below.

A concentrated solution is particularly useful for oral administration, especially for filling capsules. For parenteral use, e.g. for injection or infusion, a solution is the preferred formulation.

The amount of CPT or ECPT contained in the formulations of this invention is not specifically restricted but may be any amount convenient for pharmaceutical purposes, and may be selected according to the dosage to be prepared. A preferred capsule filling solution contains from 0.1 mg to 50.0 mg, more preferably from 0.1 mg to 10.0 mg of CPT or ECPT activity per ml of solution.

The formulations of the invention are sufficiently acidic to cause more than 50 mole % CPT or ECPT to be in the lactone form, preferably 100 mole %. Their pH will therefore be well below 7, normally from 2 to 5 and preferably from 3 to 4.

Any pharmaceutically acceptable acid may be used: for example, mineral acids such as hydrochloric acid; and organic carboxylic acids, such as tartaric, citric, succinic, fumaric, or maleic acids. An organic carboxylic acid is preferred, and citric acid is most preferred. The amount of acid used will usually be from 0.005 to 0.9 more usually 0.05 to 0.5 part by weight of acid per part by weight of CPT or ECPT, or, in another preferred embodiment 0.01 to 0.9 and preferably from 0.01 to 0.3, more preferably 0.1 to 0.3 part by weight of acid per part by weight of CPT or ECPT. Citric acid is preferably used in a proportion of from 0.01 to 0.5, especially from 0.05 to 0.1, or from 0.1 part to 0.5 by weight when taurocholic acid or a pharmaceutically acceptable salt thereof is included in the formulations, all per part by weight of CPT or ECPT.

In the formulations of the invention, CPT and ECPT are soluble and maintained in their active lactone forms. The non-enzymatic conversion of the pH labile E ring from the closed lactone (active) to the open carboxylate form (inactive) is reduced by formulating CPT or ECPT under acidic conditions (pH 2 to 5). The acid is preferably included to assure that an acidic pH value is maintained upon dilution in the stomach. It is believed that this results in the formation of a micellar solution of CPT or ECPT in the stomach which is readily absorbed by the gastrointestinal tract. Applicants do not wish to be bound, however, by any theoretical explanation of the mechanism by which the superior oral bioavailability of the present CPT or ECPT formulation is achieved. Examples of preferred solid water-soluble organic carboxylic acids effective in this invention include citric, gluconic, maleic, tartaric, or ascorbic acids. Other acids may be employed, but citric acid is most preferred.

When oral dosages are to be administered in a capsule form, it is clearly superior to have a concentrated solution of CPT or ECPT suitable for encapsulation within a soft or hard gelatin capsule. Concentrated solutions allow the preparation of capsules of smaller size which allows easier ingestion by the patient, and may also reduce the number of capsules to be swallowed. These factors are important in view of the generally poor condition of cancer patients.

Taurocholic acid, a bile acid, may enhance the intestinal absorption of the drug in certain patients. The present invention takes advantage of the discovery that taurocholic acid, or a pharmaceutically acceptable salt thereof, when included with CPT or ECPT in a composition of the invention, results in improved absorption of the drug following ingestion of the composition. It is believed that this is due to the formation of a micellar solution of CPT or ECPT on dilution thereof with the gastric contents.

The phenomenon of micellar solubilization of poorly water-soluble drugs mediated by bile acids, including taurocholic acid, has been previously reported with respect to glutethimide, hexestrol, griseofulvin (Bates et al.), reserpine (Malone et al,) and fatty acids and cholesterol (Westergaard et al.). The use of taurocholic acid or a pharmaceutically acceptable salt thereof in the present invention involves a pharmaceutical solution of CPT or ECPT which has the unique property of providing a stable solution or suspension of the drug upon dilution thereof with from 1 to 100 volumes of water. The solution or suspension is stable and free of precipitate for a period of at least two hours, sufficient time to permit administration and absorption by the patient.

### Antitumor Compositions Comprising HECPT

A preferred embodiment of the claimed invention is an antitumor composition comprising a solution or suspension of (1) CPT or ECPT, (2) DMI or DMA, and (3) from 0.01 to 0.9, preferably 0.01 to 0.2, part by weight of a pharmaceutically acceptable organic carboxylic acid, most preferably 0.05 to 0.1 part of citric acid, per part by weight of CPT or ECPT.

In another embodiment, the antitumor composition further comprises taurocholic acid or a pharmaceutically acceptable salt thereof and polyethylene glycol.

Another embodiment of the claimed invention is the antitumor composition further comprising ethanol or benzyl alcohol (as a preservative) or both. Another embodiment of the claimed invention is the antitumor composition further comprising glycerin as a co-solvent.

Yet another embodiment of this invention is wherein the antitumor composition further comprises taurocholic acid or a pharmaceutically acceptable salt thereof, polyethylene glycol, ethanol, glycerin, and a buffer, such as sodium acetate, to maintain an acid pH.

A particularly preferred antitumor composition of the invention contains for each part by weight of CPT or ECPT, 1-10 parts by weight of DMI or DMA, 0.005-0.5 parts by weight of a pharmaceutically acceptable acid, preferably an organic carboxylic acid, most preferably citric acid, 1-10 parts by weight of taurocholic acid or a pharmaceutically acceptable salt thereof and 1-10 parts by weight of polyethylene glycol. Preferably it also contains 0.1-2 parts by weight of glycerin, 0.1-2 parts by weight of ethanol and 0.005-0.5 parts of a buffer.

The polyethylene glycol preferably has a molecular weight of about 300.

In another embodiment the antitumor composition further comprises a non-ionic surfactant, preferably a poloxamer. The preferred poloxamer is PF-127.

In yet another embodiment of this invention, the antitumor composition further comprises ethyl or benzyl alcohol, polyethylene glycol, and surfactant. Preferably, the pharmaceutically acceptable organic acid is citric acid, the polyethylene glycol has a molecular weight of about 300, the alcohol is ethanol and the surfactant is polysorbate - 80.

Another embodiment of this invention is an antitumor composition comprising a solution or suspension of (1) 0.1 mg to 50.0 mg, more preferably 0.1 mg to 10.0 mg of CPT or ECPT, (2) 1 to 10 parts of DMI or DMA and (3) 0.1 to 0.5 parts of a pharmaceutically acceptable organic carboxylic acid, to adjust to a final pH of between 3 and 4. This antitumor composition further comprises 5 to 9 parts by weight of polyethylene glycol, 0.1 to 2.0 parts of a pharmaceutically acceptable alcohol, and 1 to 10 parts of a non-ionic surfactant. Here and elsewhere herein where quantities of CPT or ECPT are given in mg., "parts" means per part by weight of CPT or ECPT. Preferably the acid is citric acid, the polyethylene glycol has a molecular weight of about 300, the alcohol is ethanol and the surfactant is polysorbate - 80.

Another embodiment of this invention is an antitumor composition comprising a solution or suspension of (1) 0.1 mg to 50.0 mg, more preferably 0.1 mg to 10.0 mg of CPT or ECPT, (2) 1 to 10 parts of DMI or DMA and (3) 0.1 to 0.5 parts of a pharmaceutically acceptable organic carboxylic acid. This solution further comprises 0.1 to 2.0 parts of a pharmaceutically acceptable alcohol, and 1 to 10 parts of a non-ionic surfactant. Preferably, for this antitumor composition, the acid is citric acid, the alcohol is ethanol, and the non-ionic surfactant comprises polyoxyethylated castor oil.

Another embodiment of this invention is an antitumor composition comprising a solution or suspension of (1) 0.1 mg to 50.0 mg, more preferably 0.1 mg to 10.0 mg of CPT or ECPT, (2) 1 to 10 parts of DMI or DMA, (3) 0.1 to 0.9 parts citric acid, (4) 1 to 10 parts polyoxyethylated castor oil and (5) 0.1 to 2 parts by weight dehydrated ethyl alcohol.

In a more preferred embodiment, CPT or ECPT is solubilized in a manner suitable for clinical use by forming a sterile, nonaqueous solution of 1 part of CPT or ECPT, preferably 1.0 - 2.0 mg, in a vehicle comprising dehydrated ethyl alcohol 0.1-2.0 parts, benzyl alcohol 0.1-2.0 parts, citric acid 0.1-0.9, more preferably 0.1 to 0.5 parts, polyethylene glycol (molecular weight 200-300) 4 to 10 parts, more preferably PEG 300 5 to 9 parts, polysorbate - 80 ("Tween" 80) 1 to 10 parts, and DMI or DMA 1 to 10 parts, all parts being by weight, in acidified medium with a pH of 3 to 4. (Tween is a registered Trade Mark)

Another more preferred formulation, for dilution prior to parenteral administration, comprises 1 part (0.1 to 2.5 mg) CPT or ECPT in 2 ml of nonaqueous solvents including 1 to 10 parts "Cremaphor" EL (polyoxyethylated castor oil), 0.1 to 2 parts dehydrated ethyl alcohol, DMI or DMA 1 to 10 parts, and citric acid 0.1-0.9 parts to adjust the final pH to between 3 to 4, or possibly 0.01 to 0.5 part of citric acid, all parts being by weight.

Another embodiment of this invention is a lactone stable CPT or ECPT (a formulation of the invention) for use in administration to a patient with cancer.

For the purpose of this invention, a previously untreated patient is one who has not been previously treated for said cancer with any chemotherapeutic drugs.

### Preferred Dosages and Schedules for Parenteral Administration of Compositions of the Invention

All dosages refer to the active ingredient (CPT or ECPT) administered as the lactone stable solution or suspension, by infusion, to patients with cancer.
- 2.0 mg/m² to 33.0 mg/m² over a duration of approximately 120 minutes every 21 to 28 days.
- from 1.0 mg/m² to 16.0 mg/m² over a duration of approximately 120 minutes for three consecutive days every 21 to 28 days.
- from 1.0 mg/m² to 20.0 mg/m² over a duration of approximately 120 minutes given once per week for three consecutive weeks with 2 weeks rest after each 3 week cycle.
- to a previously untreated patient with cancer, from 2.0 mg/m² to 24.0 mg/m² over a duration of approximately 120 minutes given once per week for three consecutive weeks with two weeks rest after each 3 week cycle.
- continuous infusion of from 0.1 mg/m²/d to 6.0 mg/m²/d over a duration of approximately 24 to 120 hours every 21 to 28 days.

### Preferred Dosages and Schedules for Oral Administration of ECPT Compositions of the Invention

All dosages refer to the active ingredient (CPT or ECPT) administered orally as the lactone solution or suspension in a single dose or divided into smaller doses, to patients with cancer.
- from 2.5 mg/m² to 100 mg/m² within a 24 hour period every 21 to 28 days.
- from 1.0 mg/m² to 50 mg/m² for three consecutive days every 21 to 28 days.
- from 1.0 mg/m² to 60 mg/m² within a 24 hour period given once per week for three consecutive weeks with 2 weeks rest after each 3 week cycle.
- to a previously untreated patient, from 2.0 mg/m² to 75 mg/m² within a 24 hour period once per week for three consecutive weeks with 2 weeks rest after each 3 week cycle.
- from 0.5 mg/m²/d to 18.0 mg/m²/d administered within each 24 hour period for two to five consecutive days and repeated every 21 to 28 days.

A further embodiment of this invention is encapsulating the oral formulations within a hard or soft gelatin capsule, preferably in soft gelatin capsules (comprised of gelatin/glycerin/sorbitol/purifiers/purified water) containing 1.0 part of CPT or ECPT in a vehicle comprising citric acid 0.1 to 0.9 parts, glycerin 1 to 10 parts, polyethylene glycol (molecular weight 200 to 300) 5 to 9 parts, dehydrated ethyl alcohol 10 to 20% of total solution weight, sodium acetate 0.05 to 0.5 parts, a surfactant, and 1 to 10 parts dimethylisosorbide, all parts being by weight. A more preferred oral formulation will include as a surfactant "Pluronic" F-127 poloxamer using 0.05 to 1.0 parts by weight.

Another preferred oral formulation includes the addition of taurocholic acid 2 to 10 parts by weight. The soft gelatin capsules may also be composed of any of a number of compounds used for this purpose including, for example, a mixture of gelatin, glycerin, sorbitol, purified water, and parabens.

The table below indicates parts by weight of different components to be included in the preferred oral formulation to be administered in capsules. Four components are marked with an "**" which denotes that the components are "optional", even in this preferred formulation. For the purpose of this invention, inclusion of these components depends on a variety of different factors, i.e. type of cancer the patient has, whether pretreated previously, etc.

| Ingredients | Parts by Weight |
|---|---|
| CPT or ECPT | 1 |
| Citric Acid | 0.1-0.5 |
| Glycerin ** | 0.4-2 |
| PEG 300 | 5-9 |
| EtOH ** | 10-20% by weight of total solution weight |
| Dimethylisosorbide | 1-10 |
| Poloxamer surfactant (Pluronic F-127) ** | 0.05-1.0 |
| Sodium Acetate | 0.05-0.5 |
| Taurocholic Acid ** | 2-10 |

Clinicians will administer CPT or ECPT to human patients with cancer according to schedules that maximize its potential antitumor effects and diminish its potential toxic side effects. Except at extremely high doses which produce high plasma concentrations of the drugs, the antitumor activity of CPT-11 and CPT or ECPT can be increased by increasing the duration of exposure (time dependent) rather than increasing the dose (dose dependent) of the drug. The greater antitumor effects associated with increasing the duration of exposure are most likely related to the predominant S-phase mode of antitumor activity of CPT-11 and CPT or ECPT. CPT and ECPT are S-phase-active agents; therefore, the greater antitumor effect in humans will likely be observed with prolonged infusion or closely spaced repetitive administration schedules. Such schedules of administration would expose more cycling tumor cells to the drug and increase the frequency of exposure to the tumor cells in S-phase to sufficiently toxic levels of the drug.

The claimed formulations and compositions of the invention may be used in treatment of a number of tumors including, without limitation, human cancers of the lung, breast, colon, prostate, melanoma, pancreas, stomach (gastric), liver, brain, kidney, uterus, cervix, ovaries, and urinary tract.

The site and type of tumor to be treated will, in many cases, influence the preferred route of administration and therapeutic regimen to be applied. Consequently, although the formulations of the invention may be most usually administered by intravenous injection or infusion, they also can be delivered directly into the tumor site or by other methods designed to target the drug directly to the tumor site. For example, in patients with malignant pleural effusion, the intrapleural route may be preferred; in patients with poor venous access, the subcutaneous route of administration may be preferred; in patients with primary or metastatic cancer involving the brain or nervous system, the intracisternal or intrathecal route of administration may be most advantageous; in patients with malignant ascites secondary to cancer, one may select intraperitoneal administration; and in patients with bladder cancer direct intravesicular instillation may be most advantageous. Similarly, in tumors of the skin, the formulation may be topically applied. An oral formulation is also provided for use where suitable.

The formulations of the invention, sterilized where desirable, can be prepared for oral, intrapleural, intrathecal, subcutaneous, intracisternal, intravesicular, intraperitoneal, topical or parenteral administration to a patient with cancer.

The formulations of the invention may contain both CPT and ECPT and may also be used in conjunction with other drugs in methods of convergent therapy whereupon an additional drug or drugs are co-administered along with the lactone stable CPT or ECPT composition, e.g. with CPT-11 (preferred), topotecan, 10,11-methylenedioxy camptothecin or any combinations of two or more of these, using a pharmaceutically acceptable carrier, and the co-administration is based on an optimal dosage and schedule. For example, CPT-11 and topotecan may be co-administered with the lactone stable CPT or ECPT.

A further embodiment is the lactone stable CPT or ECPT, for use in treatment of cancer in humans by convergent therapy or combination therapy, e.g. with one or more additional drugs selected from the group consisting of, but not limited to, carmustine, azathioprine, cisplatinum, carboplatin, iproplatin, cyclophosphamide, ifosfamide, etoposide, ara-C, doxorubicin, daunorubicin, nitrogen mustard, 5-fluorouracil, bleomycin, mitomycin-C, fluoxymesterone, mechlorethamine, teniposide, hexamethylmelamine, leucovorin, melphelan, methotrexate, mercaptopurine, mitoxantrone, BCNU, CCNU, procarbazine, vincristine, vinblastine, vindesine, thioTEPA, amsacrine, G-CSF, GM-CSF, erythropoietin, γ-methylene-10-deazaaminopterin or γ-methylene-10-ethyl-10-deazaaminopterin, taxol, and 5-azacytidine. For the purpose of this invention, the terms convergent, co-administered, and combination are used interchangeably.

The formulations of this invention for use when administered parenterally, are preferred diluted with an appropriate volume of a parenteral vehicle to a concentration of about 0.1 mg/ml or lower of ECPT activity. A further embodiment of the invention is a sterile solution or suspension of any of the claimed ECPT formulations for sterile administration to a patient with cancer upon dilution with a sterile parenteral vehicle. For the purposes of this invention, parenteral vehicles include dextrose 5 to 10% in water, 0.9% NaCl in water with or without 5% or 10% Dextrose, 0.45% NaCl in water with or without 5% or 10% Dextrose, and 3% NaCl in water with or without 5% to 10% Dextrose, or sterile lipid formulations, such as intralipid, used for parenteral nutritional support for cancer patients.

The formulations of the invention can be prepared by mixing the components all at once, by first adding the CPT or BCPT to DMA or DMI and then adding the acid or by adding the acid to DMA or DMI and then adding the CPT or ECPT to the DMA/DMI-acid mixture. Various excipients, diluents, preservatives and other optional ingredients as described above may be added subsequently or with any of the essential components.

### EXAMPLES

The following examples illustrate selected modes for carrying out the claimed invention.

### EXAMPLE 1

For injection or infusion into aqueous body fluids, a formulation comprises from 0.1 to 50.0 mg, preferably 0.1 to about 10.0 mg of CPT or ECPT mixed with 1 to 10 parts of DMA in an acidified vehicle comprising between about 10 to about 40 percent of an acceptable alcohol, about 4 to about 10 parts by weight of polyether glycol, and about 1 to about 10 parts of a non-ionic surfactant. Suitable alcohols include dehydrated ethyl alcohol and benzyl alcohol. Suitable polyether glycols include polyethylene glycol 200, polyethylene glycol 300 and propylene glycol. Suitable non-ionic surfactants include polysorbate - 80. In a preferred embodiment, the formulation of CPT or ECPT is supplied for intravenous injection in a glass vial comprising a sterile, nonaqueous solution of drug in a vehicle comprising dehydrated ethyl alcohol, benzyl alcohol, citric acid, polyethylene glycol 300, and polysorbate ("Tween" 80) in acidified medium with a pH of 3 to 4 at a final concentration of 1 mg per 1 to 2 ml.

### EXAMPLE 2

A second formulation comprises from about 0.1 mg to about 50 mg, preferably 0.1 mg to about 10.0 mg of CPT or ECPT in an acidified vehicle comprising between about 0.1 to 2 parts of an alcohol and about 1 to 10 parts of a non-ionic surfactant. Suitable alcohols include dehydrated ethyl alcohol and benzyl alcohol. Suitable non-ionic surfactants include the polyoxyethylated oils, such as polyoxyethylated vegetable oils, such as castor oil, peanut oil, and olive oil. In a preferred embodiment 0.1 mg to 8.0 mg CPT or 0.1 to 10.0 mg ECPT is formulated in 1 to 10 parts of DMA, 1 to 10 parts of "Cremaphor" EL (polyoxyethylated castor oil), 0.1 to 2 parts by weight dehydrated ethyl alcohol, and 0.1 to 0.9 parts citric acid to adjust the final pH between 3 to 4.

### EXAMPLE 3

An oral formulation of CPT or ECPT in soft gelatin capsules (comprised of gelatin/glycerin/sorbitol/purifiers/purified water) containing 1.0 part of CPT or ECPT in 1 to 10 parts of DMA, citric acid 0.1 to 0.5 parts by weight, glycerin 1 to 10 parts by weight, and polyethylene glycol 200 to 300 5 to 9 parts by weight, dehydrated ethyl alcohol 0.2 to 2 parts by weight of total solution weight, sodium acetate 0.05 to 0.5 parts by weight, "Pluronic" F-127 or other acceptable poloxamer 0.05 to 1.0 parts by weight, and taurocholic acid 2 to 10 parts by weight. The soft gelatin capsules may also be composed of any of a number of compounds used for this purpose including, for example, a mixture of gelatin, glycerin, sorbitol, purified water, and parabens.

Maintaining an acidic pH as set forth above (especially 3 to 4) in the formulation is particularly important to reduce the slow conversion of HECPT lactone to the E-ring-hydrolyzed carboxylate, which occurs at physiological pH.

To further prolong the stability and solubility of CPT or ECPT for clinical infusions, the lactone stable formulation may be diluted in 5% dextrose in water (D5W) to a final concentration of 0.001 mg/ml to about 0.1 mg/ml of CPT or ECPT prior to injection or infusion.

Initially, patients may be treated in a dose escalation protocol to determine the maximal tolerated dose of the formulation. In determining a safe starting dose for CPT or ECPT, the data from Ohe et al. and Rothenberg et al. are helpful.

The administration of the lactone stable CPT or ECPT may be carried out using various schedules and dosages. For example:
1. For intravenous administration, a suitable dose is 0.1 mg to 5.4 mg/m² per day using a 3 to 5 day continuous infusion schedule every 21 to 30 days or 2.0 to 32.0 mg/m² given as a 30 to 90 minute infusion every 21 to 30 days.
2. Another schedule involves the administration of 1.0 to 16.0 mg/m² daily for three consecutive days over 90 minutes intravenously every 21 to 28 days.
3. A suitable oral dose of the drug is 0.5 to 50 mg/m² per day using the lower dose for a period of 3 to 5 days and using divided dosages of administration of two to four times per day.

In addition, patients may be given the lactone stable CPT or ECPT as an inpatient or outpatient using the following exemplary schedules:
1. 2.0 to 33.0 mg/m² given over 90 minutes I.V. every 21 to 28 days;
2. 1.0 to 16.0 mg/m² given daily for three consecutive days over 90 minutes I.V. every 21 to 28 days;
3. 1.0 to 20.0 mg/m² given once per week X 3 consecu tive weeks over 90 minutes I.V. with 2 weeks rest after each 3 week cycle for pretreated patients;
4. 2.0 to 25.0 mg/m² given once per week X 3 consecu tive weeks over 90 minutes I.V. for previously untreated patients with 2 weeks rest after each 3 week cycle; and
5. 0.1 to 6.0 mg/m²/d X 3 to 5 consecutive days as a continuous I.V. infusion every 21 to 28 days.

The words "CREMAPHOR", PLURONIC" and "TRITON" are Registered Trade Marks.

### LITERATURE REFERENCES

Bates et al., *Solubilizing Properties of Bile Salt Solutions. I. Effect of Temperature and Bile Salt Concentration on Solubilization of Glutethimide, Griseofulvin and Hexestrol*. Journal of Pharmaceutical Sciences, 55:191-199, (1966).

Bates et al., *Rates of Dissolution of Griseofulvin and Hexestrol in Bile Salt Solutions*. Chem. Abstracts 65:8680b, (1966).

Bates et al., *Solubilizing Properties of Bile Salt Solutions on Glutethimide, Griseofulvin, and Hexestrol*. Chem. Abstracts 64:9517e 1966; 65:15165a, (1966).

Malone et al., *Desoxycholic Acid Enhancement of Orally Administered Reserpine*. Journal of Pharmaceutical Sciences, 55:972-974, (1966).

Ohe, Y. et al., *Phase I Study and Pharmacokinetics of CPT-11 with 5-day Continuous Infusion*. JNCI 84(12):972-974, (1992).

Rothenberg, M.L. et al., *A Phase I and Pharmacokinetic Trial of CPT-11 in Patients with Refractory Solid Tumors*. Amer. Soc. Clin. Onc. 11:113, (1992).

Rothenberg, M.L., Kuhn, J.G., Burris, H.A., Nelson, J., Eckardt, J.R., Tristan-Morales, M., Hilsenbeck, S.G., Weiss, G.R., Smith, L.S., Rodriguez, G.I., Rock, M.K., Von Hoff, D.D. *Phase I and Pharmacokinetic Trial of Weekly CPT-11*. Journal of Clinical Oncology. 11:2194-2204, (1993).

Westergaard et al., *The Mechanism Whereby Bile Acid Micelles Increase the Rate of Fatty Acid and Cholesterol Uptake Into the Intestinal Mucosal Cell*. Journal of Clinical Investigation, 58: 97-108, (1976).

## Claims

1. A pharmaceutically acceptable formulation, in the form of an acidic solution, comprising:
(1) camptothecin (CPT) or 7-ethyl camptothecin (ECPT), more than 50 mole % of which is in the lactone form (1a'): 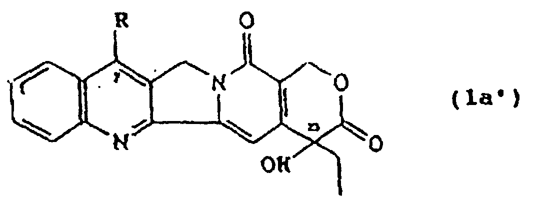 wherein R is hydrogen or ethyl,
**characterised in that** it further comprises:
(2) dimethylisosorbide (DMI) or dimethylacetamide (DMA).

2. A formulation according to Claim 1, **characterised by** a pH of from 2 to 5.

3. A formulation according to Claim 2, **characterised by** a pH of from 3 to 4.

4. A formulation according to Claim 1, 2 or 3, **characterised in that** it further comprises taurocholic acid or a pharmaceutically acceptable salt thereof and polyethylene glycol.

5. A formulation according to Claim 4, **characterised by** containing for each part by weight of CPT or ECPT, 1-10 parts by weight of DMI or DMA, 0.005-0.5 parts by weight of a pharmaceutically acceptable acid, 1-10 parts by weight of taurocholic acid or a pharmaceutically acceptable salt thereof and 1-10 parts by weight of polyethylene glycol.

6. A formulation according to Claim 4, **characterised in that** it further comprises benzyl alcohol or glycerin.

7. A formulation according to Claim 1, 2 or 3, **characterised in that** it further comprises ethanol or benzyl alcohol, polyethylene glycol, and a surfactant.

8. A formulation according to Claim 7, **characterised by** containing 0.1 mg to 50 mg, preferably 0.1 mg to 10.0 mg of CPT or ECPT, 1 to 10 parts of DMI or DMA, 0.1 to 0.5 parts of a pharmaceutically acceptable organic carboxylic acid, 5 to 9 parts by weight of polyethylene glycol, 0.1 to 2.0 parts of ethyl or benzyl alcohol, and 1 to 10 parts of a non-ionic surfactant, all parts being per part by weight of CPT or ECPT.

9. A formulation according to Claim 1, 2, 3, 4, 5, 6, 7 or 8, **characterised in that** it contains citric acid.

10. A formulation claimed in Claim 1, 2, 3, 4, 5, 6, 7, 8 or 9 in a form suitable for injection or infusion.

11. Capsules containing a formulation as claimed in Claim 1, 2, 3, 4, 5, 6, 7, 8 or 9.

12. A formulation claimed in Claim 1, 2, 3, 4, 5, 6, 7, 8, 9 or 10 or capsules claimed in Claim 11, for treatment of tumors.

## Patentansprüche

1. Pharmazeutische vertragliche Formulierung in Form einer sauren Lösung, welche umfaßt:
(1) Camptothecin (CPT) oder 7-Ethylcamptothecin (ECPT), wovon mehr als 50 Mol-% in der Lactonform (1a') vorliegen: 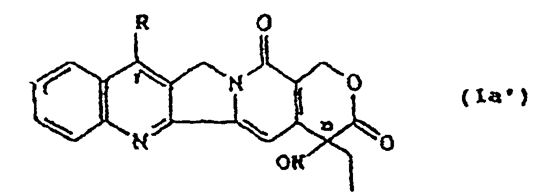 worin R Wasserstoff oder eine Ethylgruppe ist,
**dadurch gekennzeichnet, daß** sie ferner umfaßt:
(2) Dimethylisosorbid (DMI) oder Dimethylacetamid (DMA).

2. Formulierung nach Anspruch 1, **gekennzeichnet durch** einen pH-Wert von 2 bis 5.

3. Formulierung nach Anspruch 2, **gekennzeichnet durch** einen pH-Wert von 3 bis 4.

4. Formulierung nach Anspruch 1, 2 oder 3, **dadurch gekennzeichnet, daß** sie ferner Taurocholsäure oder ein pharmazeutisch verträgliches Salz davon und Polyethylenglycol umfaßt.

5. Formulierung nach Anspruch 4, **dadurch gekennzeichnet, daß** sie auf jedes Gew.-Teil CPT oder ECPT 1 bis 10 Gew.-Teile DMI oder DMA, 0,005 bis 0,5 Gew.-Teil einer pharmazeutisch verträglichen Säure 1 bis 10 Gew.-Teile Taurocholsäure oder ein pharmazeutisch verträgliches Salz davon und 1 bis 10 Gew.-Teile Polyethylenglycol enthält.

6. Formulierung nach Anspruch 4, **dadurch gekennzeichnet, daß** sie ferner Benzylalkohol oder Glycerin umfaßt.

7. Formulierung nach Anspruch 1, 2 oder 3, **dadurch gekennzeichnet, daß** sie ferner Ethanol oder Benzylalkohol, Polyethylenglycol und ein oberflächenaktives Mittel umfaßt.

8. Formulierung nach Anspruch 7, **dadurch gekennzeichnet, daß** sie 0,1 bis 50 mg, vorzugsweise 0,1 bis 10,0 mg CPT oder ECPT, 1 bis 10 Teile DMI oder DMA, 0,1 bis 0,5 Teile einer pharmazeutisch verträglichen organischen Carbonsäure, 5 bis 9 Gew.-Teile Polyethylenglycol, 0,1 bis 2,0 Teile Ethyl- oder Benzylalkohol und 1 bis 10 Teile eines nichtionischen oberflächenektiven Mittels enthält, wobei alle Teile auf Gew.-Teile CPT oder ECPT bezogen sind.

9. Formulierung nach Anspruch 1, 2 3, 4, 5, 6, 7 oder 8, **dadurch gekennzeichnet, daß** sie Citronensäure enthält.

10. Formulierung nach Anspruch 1, 2 3, 4, 5, 6, 7, 8 oder 9 in einer für die Injektion oder Infusion geeigneten Form.

11. Kapseln, die eine Formulierung nach Anspruch 1, 2 3, 4, 5, 6, 7, 8 oder 9 enthalten.

12. Formulierung nach Anspruch 1, 2 3, 4, 5, 6, 7, 8, 9 oder 10 oder Kapseln nach Anspruch 11 für die Behandlung von Tumoren.

## Revendications

1. Formulation acceptable pharmaceutiquement acceptable, se présentant sous la forme d'une solution acide, qui comprend :
(1) de la camptothécine (CPT) ou de la 7-éthylcamptothécine (ECPT), dont plus de 50 % en moles sont sous la forme de lactone (1a') : 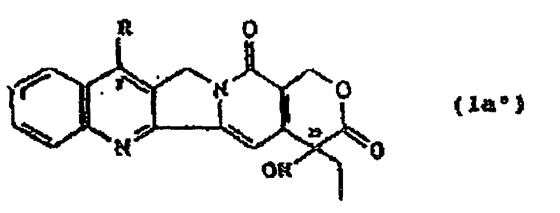 où R représente un atome d'hydrogène ou un groupe éthyle, **caractérisée par le fait qu'**elle comprend en outre
(2) du diméthylisosorbide (DMI) ou du diméthylacétamide (DMA).

2. Formulation selon la revendication 1, **caractérisée par** un pH de 2 à 5.

3. Formulation selon la revendication 2, **caractérisée par** un pH de 3 à 4.

4. Formulation selon la revendication 1, 2 ou 3, **caractérisée par le fait qu'**elle comprend en outre de l'acide taurocholique ou un sel de ce composé pharmaceutiquement acceptable et du polyéthylèneglycol.

5. Formulation selon la revendication 4, **caractérisée par le fait qu'**elle contient, pour chaque partie en poids de CPT ou d'ECPT, 1 à 10 parties en poids de DMI ou de DMA, 0,005-0,5 parties en poids d'un acide acceptable en pharmacie, 1-10 parties en poids d'acide taurocholique ou d'un sel de ce composé pharmaceutiquement acceptable et 1-10 parties en poids de polyéthylèneglycol.

6. Formulation selon la revendication 4, **caractérisée par le fait qu'**elle contient en outre de l'alcool benzylique ou de la glycérine.

7. Formulation selon la revendication 1, 2 ou 3, **caractérisée par le fait qu'**elle comprend en outre de l'éthanol ou de l'alcool benzylique, du polyéthylèneglycol et un tensioactif.

8. Formulation selon la revendication 7, **caractérisée par le fait qu'**elle contient de 0,1 mg à 50 mg, de préférence de 0,1 à 10,0 mg de CPT ou d'ECPT, de 1 à 10 parties de DMI ou de DMA, de 0,1 à 0,5 partie d'un acide organique carboxylique pharmaceutiquement acceptable, de 5 à 9 parties en poids de polyéthylèneglycol, de 0,1 à 2,0 parties d'alcool éthylique ou benzylique et de 1 à 10 parties d'un tensioactif non ionique, toutes les parties étant des parties en poids par rapport au CPT ou à l'ECPT.

9. Formulation selon la revendication 1, 2, 3, 4, 5, 6, 7 ou 8, **caractérisée par le fait qu'**elle comprend de l'acide citrique.

10. Formulation selon la revendication 1, 2, 3, 4, 5, 6, 7, 8 ou 9, sous une forme appropriée pour l'injection ou la perfusion.

11. Capsules contenant une formulation selon la revendication 1, 2, 3, 4, 5, 6, 7, 8 ou 9.

12. Formulation selon la revendication 1, 2, 3, 4, 5, 6, 7, 8, 9 ou 10, ou capsules selon la revendication 11, pour le traitement de tumeurs.
